(19) Europäisches Patentamt

European Patent Office

Office européen des brevets

(11) EP 1 731 092 A1

(12) **EUROPEAN PATENT APPLICATION**

(43) Date of publication:
**13.12.2006 Bulletin 2006/50**

(51) Int Cl.:
***A61B 5/053*** *(2006.01)*

(21) Application number: **06008662.6**

(22) Date of filing: **26.04.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **06.06.2005 JP 2005166240**

(71) Applicant: **TANITA CORPORATION**
**Tokyo**
**174-0063 (JP)**

(72) Inventors:
• **Kasahara, Yasuhiro**
  **Tokyo**
  **174-0063 (JP)**
• **Kenmochi, Hiroki**
  **Tokyo**
  **174-0063 (JP)**

(74) Representative: **Müller-Boré & Partner**
**Patentanwälte**
**Grafinger Strasse 2**
**81671 München (DE)**

(54) **Abdominal impedance-based body composition measuring apparatus**

(57)    Disclosed is an abdominal impedance-based body composition measuring apparatus, which comprises abdominal impedance measurement means for measuring an abdominal impedance, abdominal size estimation means for estimating an abdominal size, and body composition estimation means for estimating an index of body composition, based on the measured abdominal impedance and the estimated abdominal size. The abdominal impedance-based body composition measuring apparatus of the present invention makes it possible to obtain an index of body composition based on an accurate abdominal size.

# FIG. 1(a)

EP 1 731 092 A1

**Description**

BACKGROUND OF THE INVENTION

1. FIELD OF THE INVENTION

The present invention relates to an abdominal impedance-based body composition measuring apparatus for acquiring an abdominal impedance and size of the body of a user to estimate an index of body composition.

2. DESCRIPTION OF THE BACKGROUND ART

Recent years, in connection with the growing demand for fitness and beauty, research and development efforts have been made to achieve a body fat meter for estimating an index of visceral fat (e.g. visceral fat mass, visceral fat area or visceral fat volume) deeply correlated with lifestyle-related diseases, such as hypertension and hyperlipemia, and an index of abdominal subcutaneous fat (e.g. subcutaneous fat mass, subcutaneous fat area and subcutaneous fat thickness) causing adverse effects on body shape. Body fat meters as the result of such research and development have been disclosed, for example, in the following Patent Publications 1 and 2 (body fat measuring apparatus; visceral fat calculation apparatus).

The body fat meters (body fat measuring apparatus; visceral fat calculation apparatus) disclosed in the Patent Publications 1 and 2 are designed to measure an abdominal impedance using an electrode attached on the abdominal region of the body of a user, and acquire (manually enter or measure) an abdominal size [waist size (abdominal circumference, abdominal diameter)], so as to estimate indexes of visceral fat and abdominal subcutaneous fat based on the abdominal impedance and the abdominal size.

[Patent Publication 1] Japanese Patent Laid-Open Publication No. 2002-369806

[Patent Publication 2] Japanese Patent Laid-Open Publication No. 2005-103198

SUMMARY OF THE INVENTION

In the process of estimating an index of visceral fat or abdominal subcutaneous fat, the body fat meters as disclosed in the Patent Publications 1 and 2 are essentially required to acquire (manually enter or measure) an abdominal size.

However, in the type of body fat meter designed to manually enter an abdominal size, there is a low possibility that an accurate abdominal size is entered, because a user or subject rarely knows his/her accurate abdominal size in most cases. In the type of body fat meter designed to measure an abdominal size, there is a low possibility that an accurate abdominal size is measured, because the state of contact (tightening) of a member (belt) for measuring an abdominal size varies due to softness of the abdominal region.

Thus, these conventional body fat meters have a problem about low accuracy in estimating an index of visceral fat or abdominal subcutaneous fat.

In view of the above conventional problem, it is an object of the present invention to provide an abdominal impedance-based body composition measuring apparatus capable of obtaining an index of body composition based on an accurate abdominal size.

In order to achieve this object, the present invention provides an abdominal impedance-based body composition measuring apparatus which comprises abdominal impedance measurement means for measuring an abdominal impedance, abdominal size estimation means for estimating an abdominal size, and body composition estimation means for estimating an index of body composition, based on the abdominal impedance measured by the abdominal impedance measurement means and the abdominal size estimated by the abdominal size estimation means.

In the abdominal impedance-based body composition measuring apparatus of the present invention, the abdominal size estimation means may include body weight acquisition means for acquiring a body weight, body height acquisition means for acquiring a body height, BMI calculation means for calculating a BMI based on the body weight acquired by the body weight acquisition means and the body height acquired by the body height acquisition means, and abdominal size calculation means for calculating an abdominal size, based on the BMI calculated by the BMI calculation means.

In the abdominal impedance-based body composition measuring apparatus of the present invention, the abdominal size estimation means may include body weight acquisition means for acquiring a body weight, body height acquisition means for acquiring a body height, BMI calculation means for calculating a BMI based on the body weight acquired by the body weight acquisition means and the body height acquired by the body height acquisition means, and abdominal size calculation means for calculating an abdominal size, based on the BMI calculated by the BMI calculation means, and at least one of the body weight acquired by the body weight acquisition means and the body height acquired by the body height acquisition means.

In the abdominal impedance-based body composition measuring apparatus of the present invention, the abdominal size estimation means may include: body weight acquisition means for acquiring a body weight; body height acquisition means for acquiring a body height; BMI calculation means for calculating a BMI based on the body weight acquired by the body weight acquisition means and the body height acquired by the body height acquisition means; at least one selected from the group consisting of total-body-fat index acquisition means for acquiring an index of total body fat, total-lean-body index acquisition means for acquiring an index of total lean body, age acquisition means for acquiring an age and sexuality acquisition means for acquiring an sexuality; and abdominal size calculation means for calculating an abdominal size, based on the BMI calculated by the BMI calculation means, and at least one selected from the group consisting of the total body fat index acquired by the total-body-fat index acquisition means, the total lean body index acquired by the total-lean-body index acquisition means, the age acquired by the age acquisition means and the sexuality acquired by the sexuality acquisition means.

° In the abdominal impedance-based body composition measuring apparatus of the present invention, the abdominal size estimation means may include: body weight acquisition means for acquiring a body weight; body height acquisition means for acquiring a body height; BMI calculation means for calculating a BMI based on the body weight acquired by the body weight acquisition means and the body height acquired by the body height acquisition means; at least one selected from the group consisting of total-body-fat index acquisition means for acquiring an index of total body fat, total-lean-body index acquisition means for acquiring an index of total lean body, age acquisition means for acquiring an age and sexuality acquisition means for acquiring an sexuality; and abdominal size calculation means for calculating an abdominal size, based on the BMI calculated by the BMI calculation means, at least one of the body weight acquired by the body weight acquisition means and the body height acquired by the body height acquisition means, and at least one selected from the group consisting of the total body fat index acquired by the total-body-fat index acquisition means, the total lean body index acquired by the total-lean-body index acquisition means, the age acquired by the age acquisition means and the sexuality acquired by the sexuality acquisition means.

In the abdominal impedance-based body composition measuring apparatus of the present invention, the abdominal size estimation means may include body weight acquisition means for acquiring a body weight, and abdominal size calculation means for calculating an abdominal size, based on the body weight acquired by the body weight acquisition means.

In the abdominal impedance-based body composition measuring apparatus of the present invention, the abdominal size estimation means may include: body weight acquisition means for acquiring a body weight; at least one selected from the group consisting of total-body-fat index acquisition means for acquiring an index of total body fat, total-lean-body index acquisition means for acquiring an index of total lean body, age acquisition means for acquiring an age, sexuality acquisition means for acquiring an sexuality and body height acquisition means for acquiring a body height; and abdominal size calculation means for calculating an abdominal size, based on the body weight acquired by the body weight acquisition means, and at least one selected from the group consisting of the total body fat index acquired by the total-body-fat index acquisition means, the total lean body index acquired by the total-lean-body index acquisition means, the age acquired by the age acquisition means, the sexuality acquired by the sexuality acquisition means and the body height acquired by the body height acquisition means.

In the abdominal impedance-based body composition measuring apparatus of the present invention, the abdominal size may consist of either one of a waist size, an abdominal width and an abdominal depth.

In the abdominal impedance-based body composition measuring apparatus of the present invention, the index of total body fat may consist of a total body fat percentage or a total body fat mass, and the index of total lean body may consist of a total lean body percentage or a total lean body mass.

In the abdominal impedance-based body composition measuring apparatus of the present invention, the index of body composition may consist of at least one selected from the group consisting of a trunk fat percentage, a total abdominal fat area, an abdominal subcutaneous fat thickness, an abdominal muscle thickness, an abdominal subcutaneous fat area and a visceral fat area.

According to the abdominal impedance-based body composition measuring apparatus of the present invention, the abdominal impedance measurement means is operable to measure an abdominal impedance, and the abdominal size estimation means is operable to estimate an abdominal size. Then, the body composition estimation means is operable, based on the measured abdominal impedance and the estimated abdominal size, to estimate an index of body composition. In the present invention, instead of acquiring (manually entering or measuring) an abdominal size in a direct manner, the abdominal size is estimated by the abdominal size estimation means. This makes it possible to obtain an index of body composition based on an accurate abdominal size.

According to this abdominal size estimation means, the body weight acquisition means is operable to acquire a body weight, and the body height acquisition means is operable to acquire a body height. Further, the BMI calculation means is operable, based on the acquired body weight and the acquired body height, to calculate a BMI. Then, the abdominal size calculation means is operable, based on the calculated BMI, to calculate an abdominal size. A BMI is a parameter having a high correlation with an abdominal size. This makes it possible to obtain an index of body composition based on a particularly accurate abdominal size.

According to this abdominal size estimation means, the body weight acquisition means is operable to acquire a body weight, and the body height acquisition means is operable to acquire a body height. Further, the BMI calculation means is operable, based on the acquired body weight and the acquired body height, to calculate a BMI. Then, the abdominal size calculation means is operable, based on the calculated BMI and at least one of the acquired body weight and the acquired body height, to calculate an abdominal size. A combination of a BMI and at least either one of a body weight and a body height serves as a parameter having higher correlation with an abdominal size. This makes it possible to obtain an index of body composition based on a more accurate abdominal size.

According to this abdominal size estimation means, the body weight acquisition means is operable to acquire a body weight, and the body height acquisition means is operable to acquire a body height. Further, the BMI calculation means is operable, based on the acquired body weight and the acquired body height, to calculate a BMI, and at least one of the total-body-fat index acquisition means, the total-lean-body index acquisition means, the age acquisition means and the sexuality acquisition means is operable to acquire at least one of an index of total body fat, an index of total lean body, an age and an sexuality. Then, the abdominal size calculation means is operable, based on the calculated BMI and at least one of the acquired total body fat index, total lean body index, age and sexuality, to calculate an abdominal size. A combination of a BMI and at least one of a total body fat index, a total lean body index, an age and a sexuality, serves as a parameter having higher correlation with an abdominal size. This makes it possible to obtain an index of body composition based on a more accurate abdominal size.

According to this abdominal size estimation means, the body weight acquisition means is operable to acquire a body weight, and the body height acquisition means is operable to acquire a body height. Further, the BMI calculation means is operable, based on the acquired body weight and the acquired body height, to calculate a BMI, and at least one of the total-body-fat index acquisition means, the total-lean-body index acquisition means, the age acquisition means and the sexuality acquisition means is operable to acquire at least one of an index of total body fat, an index of total lean body, an age and an sexuality. Then, the abdominal size calculation means is operable, based on the calculated BMI, at least one of the acquired body weight and body height, and at least one of the acquired total body fat index, total lean body index, age and sexuality, to calculate an abdominal size. A combination of a BMI, at least one of a body weight and a body height, and at least one of a total body fat index, a total lean body index, an age and a sexuality, serves as a parameter having higher correlation with an abdominal size. This makes it possible to obtain an index of body composition based on a more accurate abdominal size.

According to this abdominal size estimation means, the body weight acquisition means is operable to acquire a body weight, and the abdominal size calculation means is operable, based on the acquired body weight, to calculate an abdominal size. A body weight is a parameter having a high correlation with an abdominal size. This makes it possible to obtain an index of body composition based on a particularly accurate abdominal size.

According to this abdominal size estimation means, the body weight acquisition means is operable to acquire a body weight, and at least one of the total-body-fat index acquisition means, the total-lean-body index acquisition means, the age acquisition means, the sexuality acquisition means and the body height acquisition means is operable to acquire at least one of an index of total body fat (preferably, a total body fat percentage or a total body fat mass), an index of total lean body (preferably, a total lean body percentage or a total lean body mass), an age, an sexuality and a body height. Then, the abdominal size calculation means is operable, based on the acquired body weight, and at least one of the acquired total body fat index (preferably, total body fat percentage or total body fat mass), total lean body index (preferably, total lean body percentage or total lean body mass), age, sexuality and body height, to calculate an abdominal size. A combination of a body weight and at least one of a total body fat index (preferably, a total body fat percentage or a total body fat mass), a total lean body index (preferably, a total lean body percentage or a total lean body mass), an age, a sexuality and a body height, serves as a parameter having higher correlation with an abdominal size. This makes it possible to obtain an index of body composition based on a more accurate abdominal size.

Each of a waist size, an abdominal width and an abdominal depth is a parameter having a high correlation with an abdominal size. This makes it possible to obtain an index of body composition based on a particularly accurate abdominal size.

BRIEF DESCRIPTION OF THE DRAWINGS

FIG 1 is an external view showing an external structure of an abdominal impedance-based body composition measuring apparatus according to one embodiment of the present invention, wherein FIGS. 1(a), 1(b) and 1(c) are, respectively, a top plan view, a front view and a side view.

FIG 2 is a block diagram showing an electric/electronic configuration of the abdominal impedance-based body composition measuring apparatus.

FIG 3 is a flowchart showing an operational process of the abdominal impedance-based body composition measuring apparatus.

FIG 4 is a schematic diagram showing the abdominal impedance-based body composition measuring apparatus during measurement of a subject.

FIG 5 is a graph showing a correlation between a waist size estimated from an abdominal size calculation formula and an actually measured waist size.

FIG 6 is a graph showing a correlation between a trunk fat percentage measured by a dual-energy X-ray absorptiometry (DXA) and a trunk fat percentage obtained from a body composition calculation formula.

FIG 7 is a graph showing a correlation between a total abdominal fat area measured by a CT scan and a total abdominal fat area obtained from a body composition calculation formula.

FIG 8 is a graph showing a correlation between an abdominal subcutaneous fat thickness measured by a CT scan and an abdominal subcutaneous fat thickness obtained from a body composition calculation formula.

FIG 9 is a graph showing a correlation between an abdominal muscle thickness measured by a CT scan and an abdominal muscle thickness obtained from a body composition calculation formula.

FIG 10 is a graph showing a correlation between an abdominal subcutaneous fat area measured by a CT scan and an abdominal subcutaneous fat area obtained from a body composition calculation formula.

FIG 11 is a graph showing a correlation between a visceral fat area measured by a CT scan and a visceral fat area obtained from a body composition calculation formula.

FIG 12 is a schematic diagram showing a screen indicating various calculation results in a display section.

FIG 13 is a diagram showing an electrical equivalent circuit model of the front abdominal region of the body of a subject.

FIG 14 is a diagram showing an electrical equivalent circuit model of the front abdominal region of the body of a subject when a current is applied thereto, wherein FIG 14(a) is an electrical equivalent model when a low-frequency current is applied thereto, and FIG 14(a) is an electrical equivalent model when a high-frequency current is applied thereto.

FIG 15 is a graph showing a correlation between a total body fat percentage measured by a DXA and a trunk fat percentage measured by the DXA.

DESCRIPTION OF THE PREFERRED EMBODIMENTS

An abdominal impedance-based body composition measuring apparatus of the present invention comprises abdominal impedance measurement means, abdominal size estimation means and body composition estimation means.

The abdominal impedance measurement means is operable to measure an abdominal impedance.

The abdominal size estimation means is operable to estimate an abdominal size (a waist size, an abdominal width, an abdominal depth, etc.). More specifically, the abdominal size estimation means is designed to calculate and estimate an abdominal size based on either one of the following structures:

(i) a first structure includes body weight acquisition means for acquiring a body weight, body height acquisition means for acquiring a body height, BMI (Body Mass Index) calculation means for calculating a BMI based on the acquired body weight and body height, and abdominal size calculation means for calculating an abdominal size, based on the calculated BMI;

(ii) a second structure includes body weight acquisition means for acquiring a body weight, body height acquisition means for acquiring a body height, BMI calculation means for calculating a BMI based on the acquired body weight and body height, and abdominal size calculation means for calculating an abdominal size, based on the calculated BMI, and at least one of the acquired body weight and body height;

(iii) a third structure includes: body weight acquisition means for acquiring a body weight; body height acquisition means for acquiring a body height; BMI calculation means for calculating a BMI based on the acquired body weight and body height; at least one selected from the group consisting of total-body-fat index acquisition means for acquiring an index of total body fat, total-lean-body index acquisition means for acquiring an index of total lean body, age acquisition means for acquiring an age and sexuality acquisition means for acquiring an sexuality; and abdominal size calculation means for calculating an abdominal size, based on the calculated BMI, and at least one selected from the group consisting of the acquired total body fat index, total lean body index, age and sexuality;

(iv) a fourth structure includes: body weight acquisition means for acquiring a body weight; body height acquisition means for acquiring a body height; BMI calculation means for calculating a BMI based on the acquired body weight and body height; at least one selected from the group consisting of total-body-fat index acquisition means for acquiring an index of total body fat, total-lean-body index acquisition means for acquiring an index of total lean body, age acquisition means for acquiring an age and sexuality acquisition means for acquiring an sexuality; and abdominal size calculation means for calculating an abdominal size, based on the calculated BMI, at least one of the acquired body weight and body height, and at least one selected from the group consisting of the acquired total body fat index, total lean body index, age and sexuality;

(v) a fifth structure includes body weight acquisition means for acquiring a body weight, and abdominal size calculation means for calculating an abdominal size, based on the acquired body weight; and

(vi) a sixth structure includes: body weight acquisition means for acquiring a body weight; at least one selected from the group consisting of total-body-fat index acquisition means for acquiring an index of total body fat, total-lean-body index acquisition means for acquiring an index of total lean body, age acquisition means for acquiring an age, sexuality acquisition means for acquiring an sexuality and body height acquisition means for acquiring a body height; and abdominal size calculation means for calculating an abdominal size, based on the acquired body weight, and at least one selected from the group consisting of the acquired total body fat index, total lean body index, age, sexuality and body height.

The body composition estimation means is operable, based on the abdominal impedance measured by the abdominal impedance measurement means and the abdominal size estimated by the abdominal size estimation means, to estimate an index of body composition (a trunk fat percentage, a total abdominal fat area, an abdominal subcutaneous fat thickness, an abdominal muscle thickness, an abdominal subcutaneous fat area, a visceral fat area, etc.).

According to the above abdominal impedance-based body composition measuring apparatus, the abdominal impedance measurement means is operable to measure an abdominal impedance, and the abdominal size estimation means is operable to estimate an abdominal size (a waist size, an abdominal width, an abdominal depth, etc.). Then, the body composition estimation means is operable, based on the measured abdominal impedance and the estimated abdominal size, to estimate an index of body composition. In the abdominal impedance-based body composition measuring apparatus, instead of acquiring (manually entering or measuring) an abdominal size in a direct manner, the abdominal size is estimated by the abdominal size estimation means. Thus, an index of body composition can be obtained based on an accurate abdominal size.

The abdominal size calculation means is operable, based on the calculated BMI or the acquired body weight, to calculate an abdominal size. A BMI or body weight is a parameter having a high correlation with an abdominal size. Thus, an index of body composition can be obtained based on a particularly accurate abdominal size.

Alternatively, the abdominal size calculation means is operable, based on the calculated BMI or the acquired body weight and at least one of the acquired total body fat index, total lean body index, age, sexuality and body height, to calculate an abdominal size. This combination serves as a parameter having higher correlation with an abdominal size. Thus, an index of body composition can be obtained based on a more accurate abdominal size.

When the abdominal size consists of either one of a waist size, an abdominal width and an abdominal depth, each of which is a parameter having a high correlation with an abdominal size, an index of body composition can be obtained based on a particularly accurate abdominal size.

An abdominal impedance-based body composition measuring apparatus according to an embodiment of the present invention will now be specifically described.

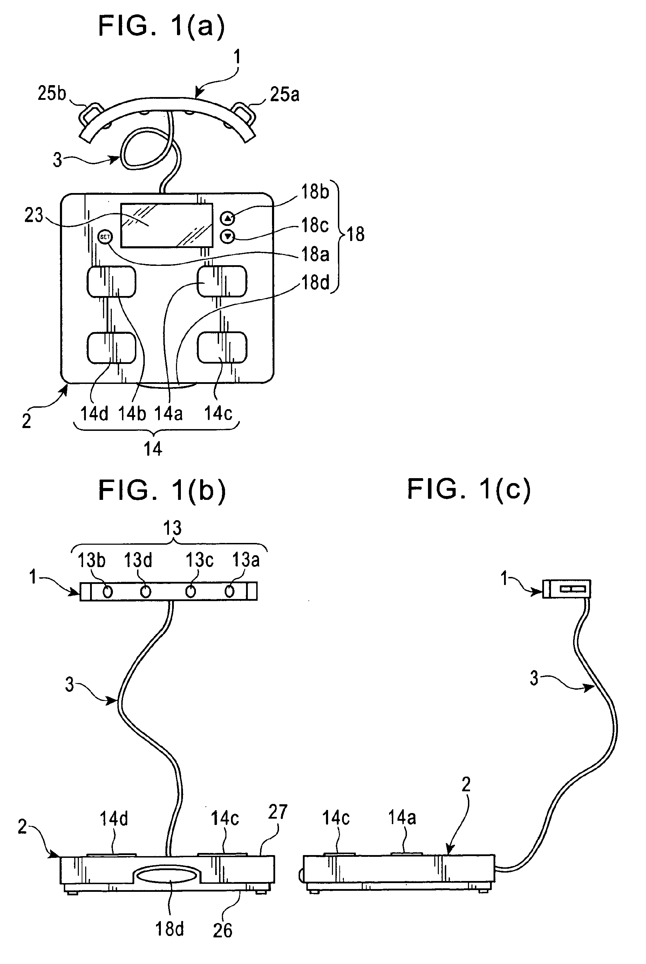

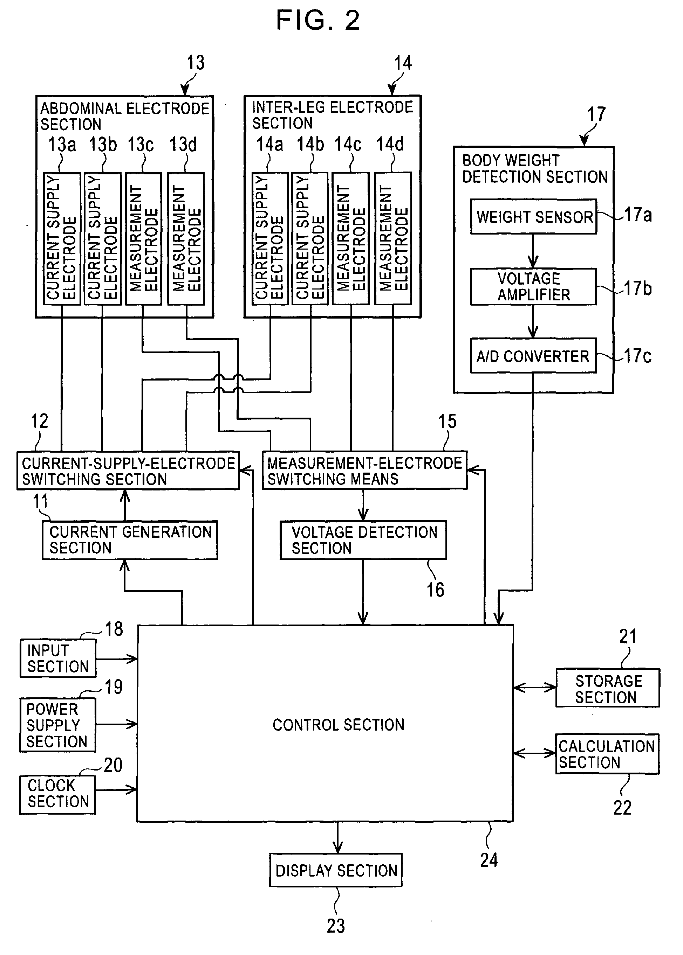

Firstly, a specific structure of the abdominal impedance-based body composition measuring apparatus will be described with reference to the external view of FIG 1 and the block diagram of FIG 2.

The abdominal impedance-based body composition measuring apparatus according to this embodiment generally comprises an abdominal unit 1, a leg unit 2, and a cord 3 electrically connecting between the abdominal unit 1 and a leg unit 2.

The abdominal unit 1 includes a U-shaped body adapted to extend along a circumference of the abdominal region of the body of a user or subject in contact manner, an abdominal electrode section 13 fixed onto an inner surface (contact surface with the abdominal region) of the U-shaped body, and a pair of grips 25a, 25a fixed onto an outer surface of the U-shaped body.

The leg unit 2 has an outer body composed of a base 26 and a mounting plate 27. The outer body houses a current generation section 11, a current-supply-electrode switching section 12, a measurement-electrode switching section 15, a voltage detection section 16, a body weight detection section 17, a power supply section 19, a clock section 20, a storage section 21, a calculation section 22, and a control section 24. The mounting plate 27 has a top surface provided with an inter-leg electrode section 14, a display section 23, and an input section 18 (18a, 18b, 18c). The base 26 has a front surface provided with an input section 18 (18d).

Each of the above sections will be described in more detail below.

The power supply section 19 is operable to supply power to each section of an electric/electronic system of this apparatus.

The input section 18 comprises a setup key 18a, an up key 18b, a down key 18c and a start key 18d, and serves as a means to allow a subject or an operator to manually enter body identification information (sexuality, body height and age) and a measurement start signal. The up key 18b and the down key 18c are used for selecting a desired one of a plurality of information items and changing a numerical value to a desired one, and the setup key 18a is used for setting up the selected information and the changed numerical value. The start key 18d is used for instructing the power supply section 19 to start supplying power so as to initiate a series of measurements.

The clock section 20 is operable to count a time.

The abdominal electrode section 13 includes two current supply electrodes 13a, 13b serving as a pair of terminals for supplying a current through the abdominal region, and two measurement electrodes 13c, 13d serving as a pair of terminals for detecting a voltage arising from the current supply.

The inter-leg electrode section 14 includes two current supply electrodes 14a, 14b serving as a pair of terminals for supplying a current through an inter-leg region (between the right and left legs), and two measurement electrodes 14c, 14d serving as a pair of terminals for detecting a voltage arising from the current supply.

The current generation section 11 is operable to selectively generate either one of a current $A_{50}$ having a basic frequency (50 kHz), a current $A_{high}$ having a high frequency (ranging from 128 kHz to 512 kHz, preferably 256 kHz) and a current $A_{low}$ having a low frequency (ranging from 4 kHz to 12.5 kHz, preferably 5 kHz), to be supplied through the abdominal region or the inter-leg region, according to control of the control section 24.

The current-supply-electrode switching section 12 is operable to switchably connect to the current supply electrodes 13a, 13b of the abdominal electrode section 13 or to the current supply electrodes 14a, 14b of the inter-leg electrode section 14, according to control of the control section 24.

The measurement-electrode switching section 15 is operable to switchably connect to the measurement electrodes 13c, 13d of the abdominal electrode section 13 or to the measurement electrodes 14c, 14d of the inter-leg electrode section 14, according to control of the control section 24.

The voltage detection section 16 is operable to detect a voltage generated in the abdominal region or the inter-leg region (a voltage $V_{50}$ to be generated based on the current $A_{50}$, a voltage $V_{high}$ to be generated based on the current $A_{high}$, a voltage $V_{low}$ to be generated based on the current $A_{low}$).

The body weight detection section 17 comprises a weight sensor 17a, a voltage amplifier 17b and an A/D converter 17c, and serves as a means to detect a voltage when a subject steps on the mounting plate 27.

The storage section 21 serves as a means to store the following calculation formulas, input information, result information, programs and various other information.

(a) A BMI calculation formula [Formula (1)] for calculating a BMI based on a body weight and a body height.
(b) A total-body-fat-percentage calculation formula [Formula (2)] for calculating a total body fat percentage based on an inter-leg impedance, a body weight, a body height, an age and a sexuality.
(c) A abdominal-size calculation formula [Formula (3)] for calculating a waist size based on a BMI, a total body fat percentage and an age.
(d) Six body-composition calculation formulas [Formulas (4) to (9)] for calculating respective indexes of six types of body compositions (trunk fat percentage, total abdominal fat area, abdominal subcutaneous fat thickness, abdominal muscle thickness, abdominal subcutaneous fat area and visceral fat area) based on an abdominal impedance (an abdominal impedance $Z_{ab50}$ generated based on the voltage $V_{50}$, an abdominal impedance $Z_{abhigh}$ generated based on the voltage $V_{high}$, an abdominal impedance $Z_{ablow}$ generated based on the voltage $V_{low}$) and a waist size.

$$\text{BMI} = \text{body weight} / (\text{body height})^2 \qquad \text{----}$$

(1)

$$\text{Total body fat percentage} = \alpha \times Z_{le50} + \beta \times \text{body weight} + \gamma \times \text{body height}$$
$$+ \delta \times \text{age} + \varepsilon \times \text{sexuality} + \xi \qquad \text{----}$$

(2)

$$\text{Waist size} = a \times \text{BMI} + b \times \text{total body fat percentage} + c \times \text{age} + d \qquad \text{----}$$

(3)

$$\text{Trunk fat percentage} = e + f \times Z_{ab50} - g \times \text{waist size} \qquad \text{----}$$

$$(4)$$

$$\text{Total abdominal fat area} = - h - i \times Z_{ab50} + j \times \text{waist size} \qquad \text{----}$$

$$(5)$$

$$\text{Abdominal subcutaneous fat thickness} = k \times Z_{ablow} + L \times \text{waist size} - m \qquad \text{----}$$

$$(6)$$

$$\text{Abdominal muscle thickness} = n \times (1 / Z_{abhigh}) + o \times (1 / \text{waist size}) - p \qquad \text{----}$$

$$(7)$$

$$\text{Abdominal subcutaneous fat area} = - q - r \times Z_{ablow} + s \times \text{waist size}$$
$$+ t \times Z_{ab50} \qquad \text{----}$$

$$(8)$$

$$\text{Visceral fat area} = - u - v \times Z_{abhigh} + w \times \text{waist size} - y \times Z_{ab50} \qquad \text{----}$$

$$(9)$$

In the above formulas,

$Z_{le50}$: inter-leg impedance,
$Z_{ab50}$, $Z_{ablow}$, $Z_{abhigh}$: abdominal impedance, and
$\alpha$ to $\xi$, a to y: coefficient (constant).

For example, in view of obtaining an adequate estimation result on an index of body composition, the coefficients a to y may be set as follows: a = 0.1; b = 1.53; c = 0.6; d = 0.1; e = 92.0; f = 0.61; g = 0.52; h = 32.1; i = 0.55; j = 1.36; k = 0.24; L = 0.035; m = 2.4; n = 38.5; o = 40.3; p = 1.9; q = 510; r = 5.63; s = 9.22; t = 202; u = 780; v = 4.3; w = 36.0; and y = 1941.

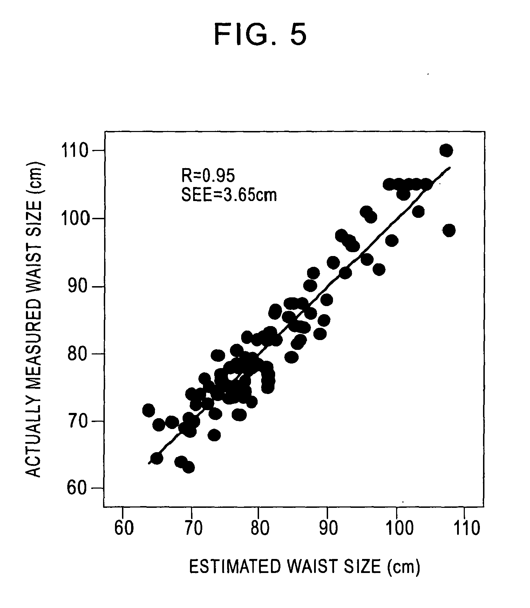

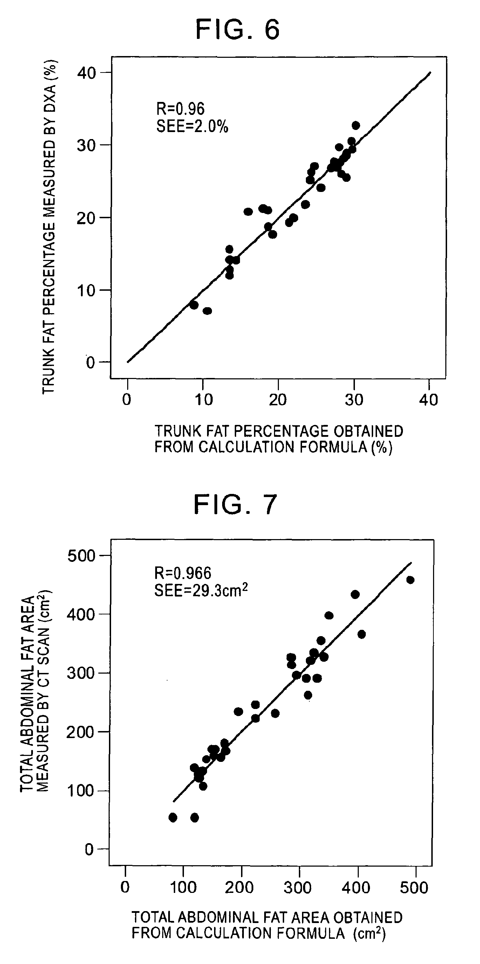

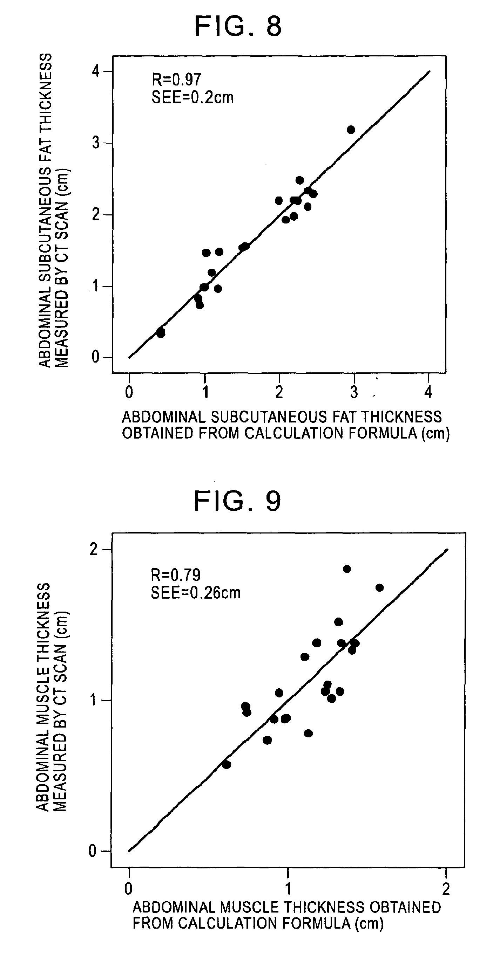

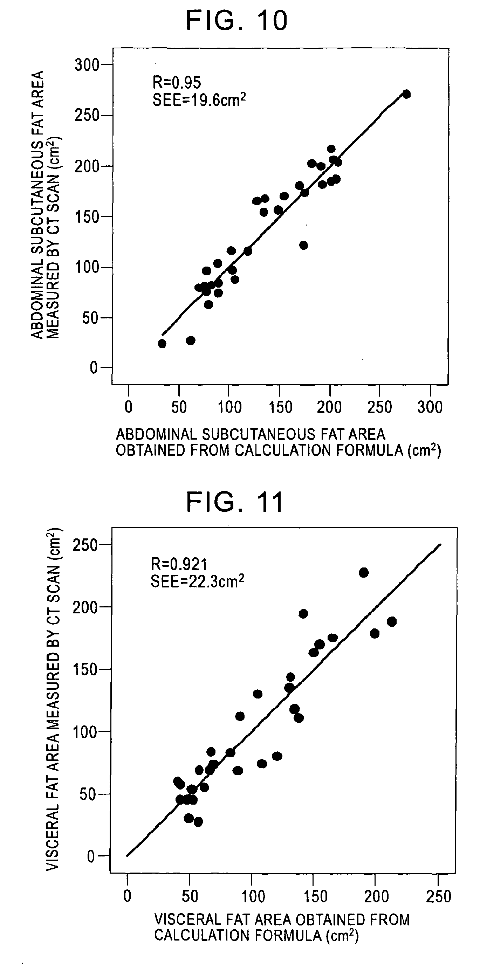

As shown in FIG 5, a waist size obtained from the above abdominal size calculation formula [Formula (3)] has a high correlation with an actually measured waist size (circumference of the abdominal region passing through the umbilicus). Further, as shown in FIGS. 7 to 11, a total abdominal fat area, an abdominal subcutaneous fat thickness, an abdominal muscle thickness, an abdominal subcutaneous fat area and a visceral fat area, which are obtained from the above body composition formulas [Formulas (5) to (9)], have high correlations, respectively, with a total abdominal fat area, an abdominal subcutaneous fat thickness, an abdominal muscle thickness, an abdominal subcutaneous fat area and a visceral fat area, which are measured by a CT (computed Tomography) scan generally considered to be excellent in estimation accuracy. As shown in FIG 6, a trunk fat percentage obtained from the above body composition formula [Formula (4)], has a high correlation with a trunk fat percentage measured by a DXA (Dual X-ray Absorptiometry) generally considered to be excellent in estimation accuracy.

The calculation section 22 is operable to perform the following calculations and other conventional calculations.

(I) A calculation of a body weight based on a voltage from the body weight detection section 17.

(II) A calculation of an inter-leg impedance ($Z_{le50}$) based on a voltage ($V_{50}$) generated between the measurement electrodes 14c, 14d of the inter-leg electrode section 14 in response to a current ($A_{50}$) supplied between the current supply electrodes 14a, 14b of the inter-leg electrode section 14.

(III) A calculation of an abdominal impedance (an abdominal impedance $Z_{ab50}$ based on a voltage $V_{50}$, an abdominal impedance $Z_{abhigh}$ based on a voltage $V_{high}$, an abdominal impedance $Z_{ablow}$ based on a voltage $V_{low}$) based on a voltage generated between the measurement electrodes 13c, 13d of the abdominal electrode section 13 (the voltage $V_{50}$ generated based on a current $A_{50}$, the voltage $V_{high}$ generated based on a current $A_{high}$, the voltage $V_{low}$ generated based on a current $A_{low}$) in response to the current ($A_{50}$, $A_{high}$, $A_{low}$) supplied between the current supply electrodes 13a, 13b of the abdominal electrode section 13.

(IV) A calculation of a BMI based on assigning the previously calculated body weight and a body height stored on the storage section 21 as input information, to the BMI calculation formula [Formula (1)] stored on the storage section 21.

(V) A calculation of a total body fat percentage based on assigning the previously calculated body weight and inter-leg impedance $Z_{le50}$, and a sexuality, body height and age which are stored on the storage section 21 as input information, to the total-body-fat-percentage calculation formula [Formula (2)] stored on the storage section 21.

(VI) A calculation of a waist size based on assigning the previously calculated BMI and total body fat percentage, and an age stored on the storage section 21 as input information, to the abdominal-size calculation formula [Formula (3)] stored on the storage section 21.

(VII) A calculation of respective indexes of body compositions (a trunk fat percentage, a total abdominal fat area, an abdominal subcutaneous fat thickness, an abdominal muscle thickness, an abdominal subcutaneous fat area and a visceral fat area) based on assigning the previously calculated abdominal impedance ($Z_{ab50}$, $Z_{abhigh}$, $Z_{ablow}$) and waist size, to the body-composition calculation formulas [Formulas (4) to (9)] stored on the storage section 21.

The display section 23 is operable to display result information about the body composition indexes (the trunk fat percentage, the total abdominal fat area, the abdominal subcutaneous fat thickness, the abdominal muscle thickness, the abdominal subcutaneous fat area and the visceral fat area) calculated by the calculation section 22.

The control section 24 is operable to perform the following controls and other conventional controls.

(A) A control of the power supply section 19 to supply power to each section of the electric/electronic system of this apparatus, in response to an ON signal from the start key 18d.

(B) A setup control of the body identification information (sexuality, body height, age) in response to an input signal from the up key 18b, the down key 18c and/or the setup key 18a.

(C) A measurement control of a body weight, an inter-leg impedance and an abdominal impedance.

(D) A control of the storage section 21 to store various information in input, measurement and evaluation stages.

(E) A control of the calculation section 22 to perform various calculations.

(F) A control of the display section 23 to display various information in measurement and evaluation stages.

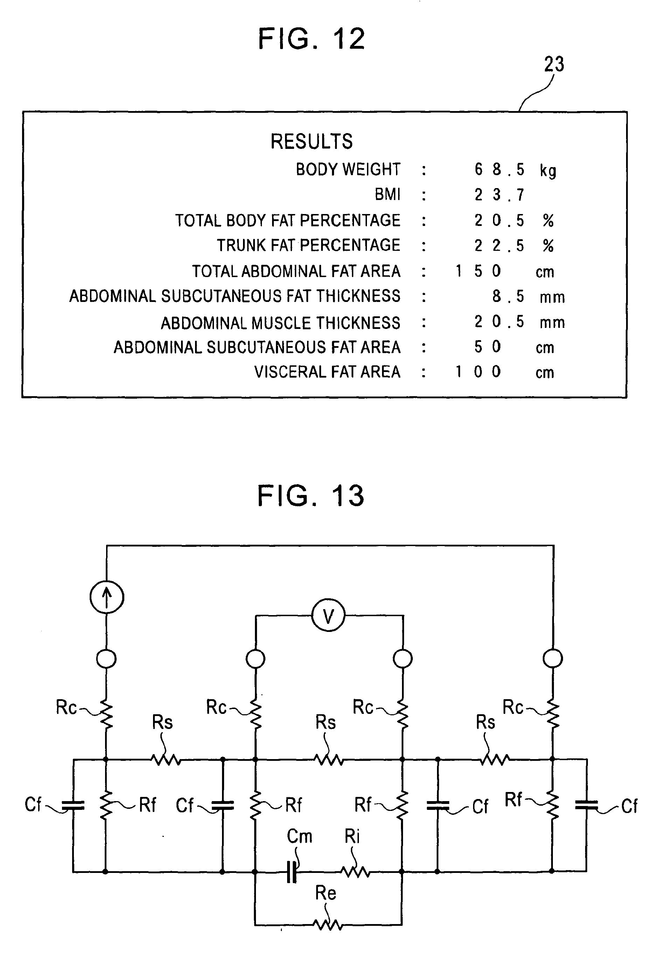

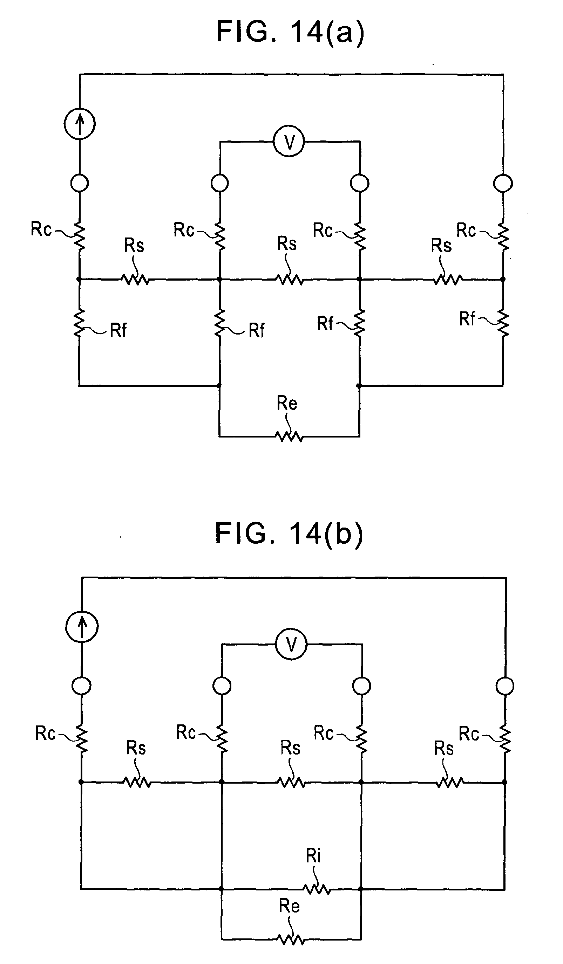

With reference to the electrical equivalent circuit models of the abdominal region illustrated in FIGS. 13 and 14, the principle of estimating an index of body composition based on measurement of an abdominal impedance will be described. Superficial tissues of the front abdominal region can be expressed as an electrical equivalent circuit model in which a skin surface is replaced by Rs; a subcutaneous fat layer is replaced by Rf and Cf; and a cell membrane, an intracellular fluid and an extracellular fluid in an abdominal muscle layer are replaced, respectively, by Cm, Ri and Re. The superficial tissues during the measurement can be expressed as an electrical equivalent circuit model prepared by adding a contact impedance Rc between the skin surface and the electrodes (the current supply electrodes and the measurement electrodes). As shown in FIG 14(a), when a low-frequency current is supplied, a current in the subcutaneous fat layer flows through only Rf without flowing through Cf, and a current in the abdominal muscle layer flows through only Re without flowing through Cf (therefore no current flows through Ri). Thus, an abdominal impedance reflecting the subcutaneous fat layer can be obtained. As shown in FIG 14(b), when a high-frequency (including the basic frequency) current is supplied, a current in the subcutaneous fat layer flows through Cf (therefore no current flows through Rf), and a current in the abdominal muscle layer flows through Cm and through Ri and Re. Thus, an abdominal impedance reflecting the abdominal muscle layer (the abdominal muscle is thinly stretched along with increase in visceral fat store and thereby an abdominal impedance will reflect visceral fat tissues) can be obtained. When a basic-frequency current is supplied, a current flows through Ri and Re, as in the case of supplying the high-frequency current. Thus, an abdominal impedance reflecting tissues other than fat tissues in a wide range (i.e. an abdominal impedance reflecting only fat tissues after eliminating tissues other than fat tissues from the entire tissues) can be obtained. Therefore, when the low-frequency current is supplied, an abdominal subcutaneous fat thickness and an abdominal subcutaneous fat area can be estimated. When the high-frequency current is supplied, an abdominal muscle thickness and a visceral fat area can be estimated. Further, when the basic-frequency current is supplied, a trunk fat percentage and a total abdominal fat area can be estimated.

A combination of the abdominal electrode section 13, the current-supply-electrode switching section 12, the measurement-electrode switching section 15, the current generation section 11, the voltage detection section 16, the calculation section 22, the storage section 21, the control section 24, the clock section 20 and the power source section 19 serves as the abdominal impedance measurement means. A combination of the body weight detection section 17, the calculation section 22, the storage section 21, the control section 24, the clock section 20 and the power supply section 19 serves as the body weight acquisition means. A combination of the input section 18, the storage section 21, the control section 24 and the power supply section 19 serves as the body height acquisition means, the sexuality acquisition means and the age acquisition means. A combination of the calculation means 22, the storage section 21, the control section 24 and the power supply section 19 serves as the BMI calculation means, the abdominal size calculation means and the body composition estimation means. A combination of the inter-leg electrode section 14, the current-supply-electrode switching section 12, the measurement-electrode switching section 15, the current generation section 11, the voltage detection section 16, the calculation section 22, the storage section 21, the control section 24, the clock section 20 and the power source section 19 serves as the total-body-fat index acquisition means.

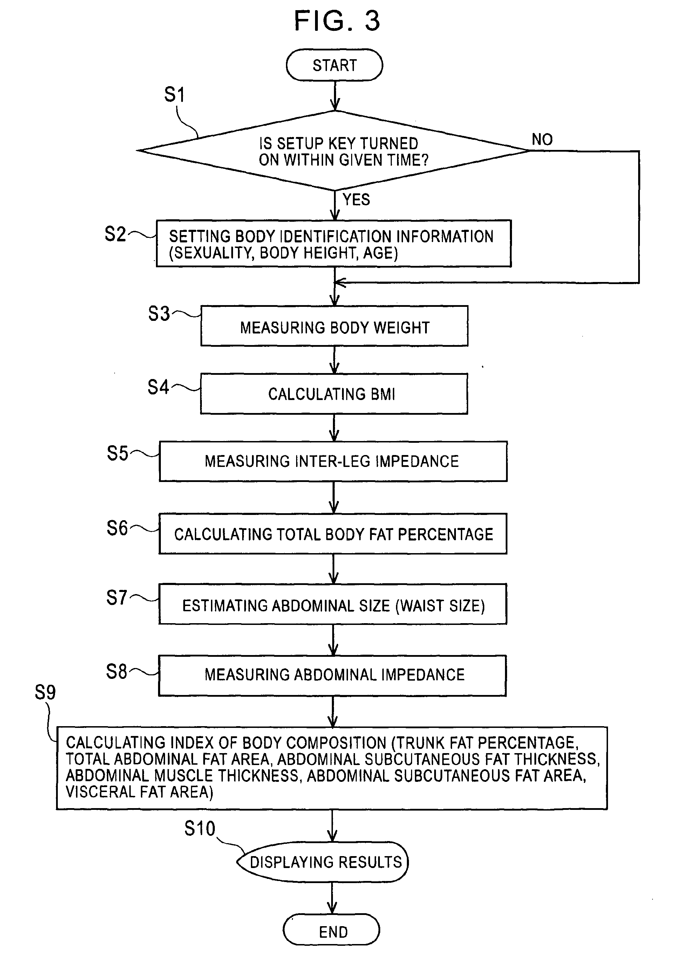

With reference to the flowchart illustrated in FIG 3, an operational process of the abdominal impedance-based body composition measuring apparatus according to this embodiment will be described below.

When the start key 18b is turned on, a power is supplied from the power supply 19 to each section of the electric/electronic system, and a body identification information (sexuality, body height, age) is displayed on the display section 23. Then, based on a time counted by the clock section 20, the control section 24 determines whether the setup key 18a is turned on within a given time (e.g. 5 seconds) (Step S1).

When the setup key 18a is turned on within the given time (YES in Step S1), a cursor appears at a position of either one of sexuality, body height and age to allow a numerical number of a pointed one of sexuality, body height and age to be changed and set up by the up key 18b, the down key 18c, the setup key 18a. When a user sets up his/her sexuality, body height and age, the storage section 21 stores the sexuality, the body height and the age (When no data is previously set up, the data is newly stored. If there is previous data, updating is performed.) (Step S2). If the setup key 18a is not turned on within the given time (NO in Step S1), the step of setting up sexuality, body height and age will be skipped (the sexuality, body height and age stored on the storage section 21 are not updated).

Then, when the setup key 18a is not turned on within the given time (NO in Step S1) or after the setup of sexuality, body height and age is completed (Step S2), a body weight measurement information ("00 kg" before measurement) is displayed on the display section 23, and the apparatus is put in a standby state of a body weight measurement. Then, when the user or subject steps on the mounting plate 27 (on the current supply electrodes 14a, 14b and the measurement electrodes 14c, 14d of the inter-leg electrode section 14), the calculation section 22 calculates a body weight based on a voltage detected by the body weight detection section 17, and the display section 23 indicates the calculated body weight (Step S3).

Then, the calculation section 22 assigns the calculated body weight and the body height stored on the storage section 21 to the BMI calculation formula [Formula (1)] stored on the storage section 21 so as to calculate a BMI (Step S4).

Then, an inter-leg impedance is measured. Specifically, the control section 24 instructs the current-supply-electrode switching section 12 and the measurement-electrode switching section 15 to switchingly connect to the current supply electrodes 14a, 14b and the measurement electrodes 14c, 14d of the inter-leg electrode section 14. Then, the current generation section 11 generates a current ($A_{50}$) to be supplied to the inter-leg region, and the voltage detection section 16 detects a voltage ($V_{50}$) generated in the inter-leg region. Then, the calculation section 22 calculates an inter-leg impedance ($Z_{le50}$) based on the detected voltage ($V_{50}$) (Step S5).

Then, the calculation section 22 assigns the previously calculated body weight and inter-leg impedance ($Z_{le50}$), and the sexuality, body height and age stored on the storage section 21, to the total-body-fat-percentage calculation formula [Formula (2)] stored on the storage section 21 so as to calculate a total body fat percentage (Step S6).

Then, the calculation section 22 assigns the previously calculated BMI and total body fat percentage, and the age stored on the storage section 21, to the abdominal-size calculation formula [Formula (3)] stored on the storage section 21 so as to calculate a waist size (Step S7).

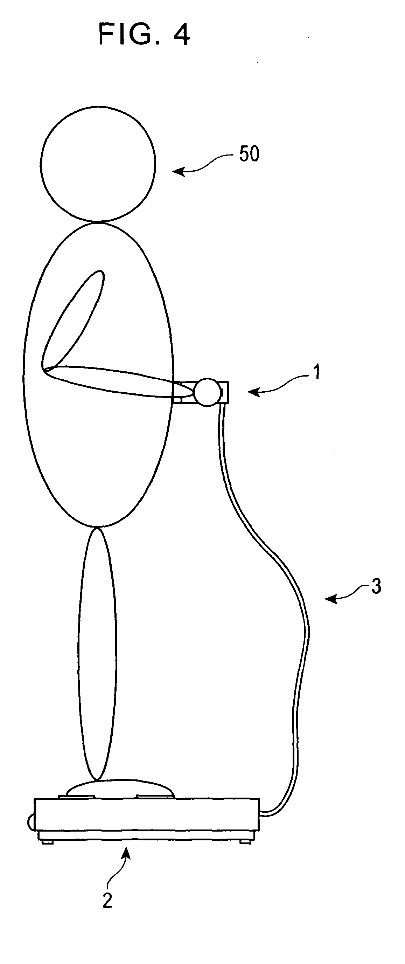

Then, an abdominal impedance is measured. As shown in FIG 4, this measurement is performed in such a manner that the inner surface (the current supply electrodes 13a, 13b and the measurement electrodes 13c, 13d of the abdominal electrode section 13) of (the U-shaped body of) the abdominal unit 1 is in contact with the abdominal region [waist (circumference of the abdominal region passing through the umbilicus)] of the subject 50. More specifically, the control section 24 instructs the current-supply-electrode switching section 12 and the measurement-electrode switching section 15 to switchingly connect to the current supply electrodes 13a, 13b and the measurement electrodes 13c, 13d of the abdominal electrode section 13. Then, the current generation section 11 generates a current ($A_{50}$, $A_{high}$, $A_{low}$) to be supplied to the abdominal region, and the voltage detection section 16 detects a voltage ($V_{50}$, $V_{high}$, $V_{low}$) generated in the abdominal region. Then, the calculation section 22 calculates an abdominal impedance ($Z_{ab50}$, $Z_{abhigh}$, $Z_{ablow}$) based on the detected voltage ($V_{50}$, $V_{high}$, $V_{low}$) (Step S8).

Then, the calculation section 22 assigns the previously calculated abdominal impedance ($Z_{ab50}$, $Z_{abhigh}$, $Z_{ablow}$) and waist size, to the body-composition calculation formulas [Formulas (4) to (9)] stored on the storage section 21 so as to calculate respective indexes of body compositions (a trunk fat percentage, a total abdominal fat area, an abdominal subcutaneous fat thickness, an abdominal muscle thickness, an abdominal subcutaneous fat area and a visceral fat area) (Step S9).

Then, the display section 23 indicates the values of body weight, BMI, total body fat percentage and body composition indexes (trunk fat percentage, total abdominal fat area, abdominal subcutaneous fat thickness, abdominal muscle thickness, abdominal subcutaneous fat area and visceral fat area) calculated by the calculation section 22, as shown in FIG. 12, and the operational process is completed.

While the abdominal impedance-based body composition measuring apparatus according to the above embodiment is designed to perform the measurement in such a manner that a subject steps on the leg unit 2 and brings the abdominal unit 1 into contact with the abdominal region while holding the abdominal unit 1 by hands, the present invention is not limited to this specific embodiment. For example, (i) two or more current supply electrodes and two or more measurement electrodes may be provided to the grips 25a, 25b of the abdominal unit 1 in the above embodiment to measure an inter-hand impedance (impedance between the right and left hands), or to measure an impedance between the hand and the leg in combination with the current supply electrodes 14a, 14b and the measurement electrodes 14c, 14d of the inter-leg electrode section 14. (ii) The abdominal impedance-based body composition measuring apparatus may comprise a hand/leg unit formed by integrating a hand unit (having two or more current supply electrodes and two or more measurement electrodes for measuring an impedance between the right and left hands or between the hand and the leg) with the leg unit 2 in the above embodiment, and a belt-type abdominal unit designed to be attached onto the abdominal region by a belt. (iii) The abdominal unit 1 in the above embodiment may be provided with the display section 23, the input section 18, the current generation section 11, the current-supply-electrode switching section 12, the measurement-electrode switching section 15, the voltage detection section 16, the power supply section 19, the clock section 20, the storage section 21, the calculation section 22 and the control section 24. In this case, the abdominal impedance-based body composition measuring apparatus may consist only of the abdominal unit designed to allow a user to enter his/her body weight from the input section 18.

Further, while the abdominal impedance-based body composition measuring apparatus according to the above embodiment is designed to estimate a waist size as an abdominal size, it may be an abdominal width or an abdominal depth to obtain the same effect.

While the abdominal impedance-based body composition measuring apparatus according to the above embodiment is designed to estimate an abdominal size based on a BMI, a total body fat percentage and an age, the present invention is not limited to this manner. For example, an abdominal size may be estimated (I) based on only a BMI, or (II) based on a BMI, and at least one selected from the group consisting of an index of total body fat (total body fat percentage, total body fat mass, etc.), a body weight, a body height, an age and a sexuality, or (III) based on only a body weight, or (IV) based on a body weight, and at least one selected from the group consisting of an index of total body fat (total body fat percentage, total body fat mass, etc.), an index of total lean body (total lean body percentage, total lean body mass, etc.), a body height, an age and a sexuality, to obtain the same effect.

Further, in an operation for obtaining an index of total lean body (total lean body percentage, total lean body mass, etc.), the total-lean-body index acquisition means may be achieved by a combination of the inter-leg electrode section 14, the current-supply-electrode switching section 12, the measurement-electrode switching section 15, the current generation section 11, the voltage detection section 16, the calculation section 22, the storage section 21, the control section 24, the clock section 20 and the power supply section 19.

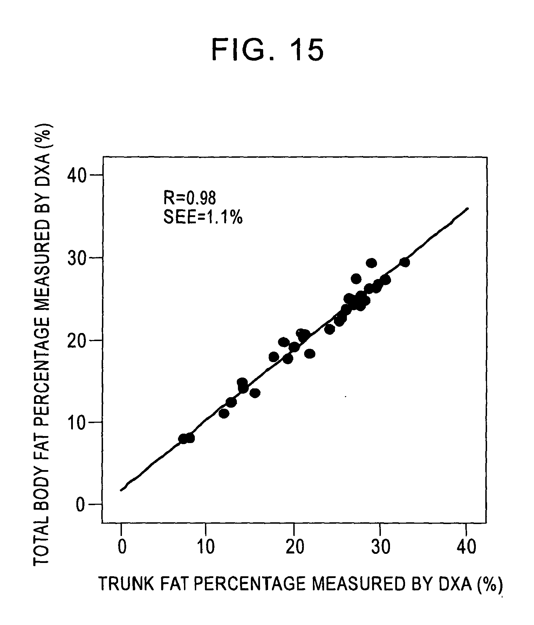

The abdominal impedance-based body composition measuring apparatus according to the above embodiment is designed to calculate a total body fat percentage based on an inter-leg impedance, because the total body fat percentage is necessary to estimate an abdominal size (waist size). As shown in FIG 15, a total body fat percentage has a high correlation with a trunk fat percentage measured by a DXA generally considered to be excellent in estimation accuracy. Thus, as an alternative, an abdominal size (waist size) may be estimated without using a total body fat percentage, and a trunk fat percentage estimated based on this estimated abdominal size (waist size) may be assigned to the following calculation formula [Formula (10)] to obtain a total body fat percentage.

$$\text{Total body fat percentage} = Q \times \text{trunk fat percentage} + R \qquad \text{---- (10)}$$

In the above formula,

Q, R: coefficient (constant).

For example, in view of obtaining an adequate estimation result on a total body fat percentage, Q and R may be set as follows: Q = 0.85 and R = 1.1.

**Claims**

1. An abdominal impedance-based body composition measuring apparatus comprising:

abdominal impedance measurement means for measuring an abdominal impedance;
abdominal size estimation means for estimating an abdominal size; and
body composition estimation means for estimating an index of body composition, based on the abdominal impedance measured by said abdominal impedance measurement means and the abdominal size estimated by said abdominal size estimation means.

2. The abdominal impedance-based body composition measuring apparatus as defined in claim 1, wherein said abdominal size estimation means includes:

body weight acquisition means for acquiring a body weight;
body height acquisition means for acquiring a body height;
BMI calculation means for calculating a BMI based on the body weight acquired by said body weight acquisition means and the body height acquired by said body height acquisition means; and
abdominal size calculation means for calculating an abdominal size, based on the BMI calculated by said BMI calculation means.

3. The abdominal impedance-based body composition measuring apparatus as defined in claim 1, wherein said abdominal size estimation means includes:

body weight acquisition means for acquiring a body weight;
body height acquisition means for acquiring a body height;
BMI calculation means for calculating a BMI based on the body weight acquired by said body weight acquisition means and the body height acquired by said body height acquisition means; and
abdominal size calculation means for calculating an abdominal size, based on:

the BMI calculated by said BMI calculation means; and
at least one of the body weight acquired by said body weight acquisition means and the body height acquired by said body height acquisition means.

4. The abdominal impedance-based body composition measuring apparatus as defined in claim 1, wherein said abdominal size estimation means includes:

body weight acquisition means for acquiring a body weight; and
abdominal size calculation means for calculating an abdominal size, based on the body weight acquired by said body weight acquisition means.

5. The abdominal impedance-based body composition measuring apparatus as defined in claim 1, wherein said abdominal size estimation means includes:

body weight acquisition means for acquiring a body weight;
body height acquisition means for acquiring a body height;
BMI calculation means for calculating a BMI based on the body weight acquired by said body weight acquisition means and the body height acquired by said body height acquisition means;
at least one selected from the group consisting of total-body-fat index acquisition means for acquiring an index of total body fat, total-lean-body index acquisition means for acquiring an index of total lean body, age acquisition means for acquiring an age and sexuality acquisition means for acquiring an sexuality; and

abdominal size calculation means for calculating an abdominal size, based on:

> the BMI calculated by said BMI calculation means; and
> at least one selected from the group consisting of the total body fat index acquired by said total-body-fat index acquisition means, the total lean body index acquired by said total-lean-body index acquisition means, the age acquired by said age acquisition means and the sexuality acquired by the sexuality acquisition means.

6. The abdominal impedance-based body composition measuring apparatus as defined in claim 1, wherein said abdominal size estimation means includes:

> body weight acquisition means for acquiring a body weight;
> body height acquisition means for acquiring a body height;
> BMI calculation means for calculating a BMI based on the body weight acquired by said body weight acquisition means and the body height acquired by said body height acquisition means;
> at least one selected from the group consisting of total-body-fat index acquisition means for acquiring an index of total body fat, total-lean-body index acquisition means for acquiring an index of total lean body, age acquisition means for acquiring an age and sexuality acquisition means for acquiring an sexuality; and
> abdominal size calculation means for calculating an abdominal size, based on:

>> the BMI calculated by said BMI calculation means;
>> at least one of the body weight acquired by said body weight acquisition means and the body height acquired by said body height acquisition means; and
>> at least one selected from the group consisting of the total body fat index acquired by said total-body-fat index acquisition means, the total lean body index acquired by said total-lean-body index acquisition means, the age acquired by said age acquisition means and the sexuality acquired by the sexuality acquisition means.

7. The abdominal impedance-based body composition measuring apparatus as defined in claim 1, wherein said abdominal size estimation means includes:

> body weight acquisition means for acquiring a body weight;
> at least one selected from the group consisting of total-body-fat index acquisition means for acquiring an index of total body fat, total-lean-body index acquisition means for acquiring an index of total lean body, age acquisition means for acquiring an age, sexuality acquisition means for acquiring an sexuality and body height acquisition means for acquiring a body height; and
> abdominal size calculation means for calculating an abdominal size, based on:

>> the body weight acquired by said body weight acquisition means; and
>> at least one selected from the group consisting of the total body fat index acquired by said total-body-fat index acquisition means, the total lean body index acquired by said total-lean-body index acquisition means, the age acquired by said age acquisition means, the sexuality acquired by the sexuality acquisition means and the body height acquired by said body height acquisition means.

8. The abdominal impedance-based body composition measuring apparatus as defined in either one of claims 5 to 7, wherein said index of total body fat consists of a total body fat percentage or a total body fat mass, and said index of total lean body consists of a total lean body percentage or a total lean body mass.

9. The abdominal impedance-based body composition measuring apparatus as defined in either one of claims 1 to 8, wherein said abdominal size consists of either one of a waist size, an abdominal width and an abdominal depth.

10. The abdominal impedance-based body composition measuring apparatus as defined in either one of claims 1 to 9, wherein said index of body composition consists of at least one selected from the group consisting of a trunk fat percentage, a total abdominal fat area, an abdominal subcutaneous fat thickness, an abdominal muscle thickness, an abdominal subcutaneous fat area and a visceral fat area.

## FIG. 1(a)

## FIG. 1(b)

## FIG. 1(c)

# FIG. 2

# FIG. 3

START

S1 — IS SETUP KEY TURNED ON WITHIN GIVEN TIME? — NO

YES

S2 — SETTING BODY IDENTIFICATION INFORMATION (SEXUALITY, BODY HEIGHT, AGE)

S3 — MEASURING BODY WEIGHT

S4 — CALCULATING BMI

S5 — MEASURING INTER-LEG IMPEDANCE

S6 — CALCULATING TOTAL BODY FAT PERCENTAGE

S7 — ESTIMATING ABDOMINAL SIZE (WAIST SIZE)

S8 — MEASURING ABDOMINAL IMPEDANCE

S9 — CALCULATING INDEX OF BODY COMPOSITION (TRUNK FAT PERCENTAGE, TOTAL ABDOMINAL FAT AREA, ABDOMINAL SUBCUTANEOUS FAT THICKNESS, ABDOMINAL MUSCLE THICKNESS, ABDOMINAL SUBCUTANEOUS FAT AREA, VISCERAL FAT AREA)

S10 — DISPLAYING RESULTS

END

# FIG. 4

# FIG. 5

# FIG. 6

# FIG. 7

## FIG. 8

## FIG. 9

# FIG. 10

R=0.95
SEE=19.6cm²

ABDOMINAL SUBCUTANEOUS FAT AREA MEASURED BY CT SCAN (cm²)

ABDOMINAL SUBCUTANEOUS FAT AREA
OBTAINED FROM CALCULATION FORMULA (cm²)

# FIG. 11

R=0.921
SEE=22.3cm²

VISCERAL FAT AREA MEASURED BY CT SCAN (cm²)

VISCERAL FAT AREA OBTAINED FROM
CALCULATION FORMULA (cm²)

# FIG. 12

23

```
                        RESULTS
                    BODY WEIGHT  :     6 8 . 5  kg
                            BMI  :     2 3 . 7
        TOTAL BODY FAT PERCENTAGE  :     2 0 . 5  %
            TRUNK FAT PERCENTAGE  :     2 2 . 5  %
          TOTAL ABDOMINAL FAT AREA  :   1 5 0    cm
ABDOMINAL SUBCUTANEOUS FAT THICKNESS  :       8 . 5  mm
      ABDOMINAL MUSCLE THICKNESS  :     2 0 . 5  mm
  ABDOMINAL SUBCUTANEOUS FAT AREA  :       5 0    cm
            VISCERAL FAT AREA  :     1 0 0    cm
```

# FIG. 13

# FIG. 14(a)

# FIG. 14(b)

# FIG. 15

R=0.98
SEE=1.1%

TRUNK FAT PERCENTAGE MEASURED BY DXA (%)

TOTAL BODY FAT PERCENTAGE MEASURED BY DXA (%)

European Patent
Office

**EUROPEAN SEARCH REPORT**

Application Number

EP 06 00 8662

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/097081 A1 (MASUDA YASUTOSHI ET AL) 22 May 2003 (2003-05-22) * figure 3 * * abstract * * paragraphs [0003], [0009], [0010], [0012] - [0015], [0029] - [0034], [0036], [0080], [0154], [0160], [0161], [0175], [0191], [0210] * | 1-10 | INV. A61B5/053 |
| A | US 2005/107717 A1 (YAMAMOTO KOJI ET AL) 19 May 2005 (2005-05-19) * abstract * | 1-10 | |
| A | EP 1 063 500 A (TANITA CORPORATION) 27 December 2000 (2000-12-27) * abstract; figure 1 * | 1-10 | |
| A | EP 1 203 562 A (TANITA CORPORATION) 8 May 2002 (2002-05-08) * abstract * * paragraphs [0014], [0015] * | 10 | |
| A | EP 1 472 977 A (TANITA CORPORATION) 3 November 2004 (2004-11-03) * abstract; figure 3 * * paragraph [0067] * | 10 | **TECHNICAL FIELDS SEARCHED (IPC)** A61B G01G |
| X | PATENT ABSTRACTS OF JAPAN vol. 2000, no. 09, 13 October 2000 (2000-10-13) & JP 2000 152919 A (YA MAN LTD), 6 June 2000 (2000-06-06) * abstract * | 1 | |

-/--

The present search report has been drawn up for all claims

| Place of search | Date of completion of the search | Examiner |
|---|---|---|
| The Hague | 27 July 2006 | Schwenke, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 06 00 8662

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PATENT ABSTRACTS OF JAPAN vol. 2002, no. 10, 10 October 2002 (2002-10-10) & JP 2002 159461 A (YAMATO SCALE CO LTD), 4 June 2002 (2002-06-04) * abstract * ----- | 1 | |
| | | | TECHNICAL FIELDS SEARCHED (IPC) |

The present search report has been drawn up for all claims

| Place of search | Date of completion of the search | Examiner |
|---|---|---|
| The Hague | 27 July 2006 | Schwenke, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 00 8662

This annex lists the patent family members relating to the patent documents cited in  the above-mentioned European search report.
The members are as contained in the European Patent Office EDP file on
The European Patent Office is in no way  liable for these particulars which are merely  given for the purpose of information.

27-07-2006

| Patent document cited in search report | | Publication date | Patent family member(s) | | Publication date |
|---|---|---|---|---|---|
| US 2003097081 | A1 | 22-05-2003 | AU | 760012 B2 | 08-05-2003 |
| | | | AU | 9597401 A | 06-05-2002 |
| | | | CA | 2397780 A1 | 02-05-2002 |
| | | | CN | 1394127 A | 29-01-2003 |
| | | | CN | 1698538 A | 23-11-2005 |
| | | | EP | 1329191 A1 | 23-07-2003 |
| | | | WO | 0234132 A1 | 02-05-2002 |
| | | | TW | 229596 B | 21-03-2005 |
| US 2005107717 | A1 | 19-05-2005 | CN | 1602804 A | 06-04-2005 |
| | | | JP | 2005103198 A | 21-04-2005 |
| EP 1063500 | A | 27-12-2000 | DE | 60014509 D1 | 11-11-2004 |
| | | | DE | 60014509 T2 | 13-10-2005 |
| | | | TW | 514510 B | 21-12-2002 |
| | | | US | 6487445 B1 | 26-11-2002 |
| EP 1203562 | A | 08-05-2002 | CN | 1350830 A | 29-05-2002 |
| | | | US | 2002052697 A1 | 02-05-2002 |
| EP 1472977 | A | 03-11-2004 | JP | 2004329412 A | 25-11-2004 |
| | | | US | 2004220492 A1 | 04-11-2004 |
| JP 2000152919 | A | 06-06-2000 | JP | 3449932 B2 | 22-09-2003 |
| JP 2002159461 | A | 04-06-2002 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002369806 A
- JP 2005103198 A